# EUROPÄISCHE PATENTANMELDUNG

(11) **EP 1 779 926 A2**
(43) Veröffentlichungstag der Anmeldung: **02.05.2007**
(21) Anmeldenummer: 06022195.9
(22) Anmeldetag: 24.10.2006
(51) Int. Cl.: B01J 20/32, A61M 1/36, B01D 15/00

(54) **Extrakorporales Blut- oder Plasmareinigungssystem**

(30) Priorität: 24.10.2005 AT 17302005
(71) Anmelder: Universität für Weiterbildung Krems, 3500 Krems (AT)
(72) Erfinder: Hellevuo, Kaisa, Dr., 3504 Krems/Stein/Donau (AT); Falkenhagen, Dieter, Univ.-Prof. Dr., 3500 Krems/Donau (AT); Weber, Viktoria, Dr., 3504 Krems/Stein/Donau (AT)
(74) Vertreter: Schwarz, Albin

(57) **Zusammenfassung**

Die vorliegende Erfindung stellt ein extrakorporales Blut- oder Plasmareinigungssystem bereit, welches ein für das Tyro3-Rezeptorprotein spezifisches Adsorptionsmittel und/oder ein für das Gas6-Protein spezifisches Adsorptionsmittel und/oder ein für Komplementfaktor B spezifisches Adsorptionsmittel umfasst. Dieses System kann zum Absenken der Konzentration an Tyro3-Rezeptorprotein und/oder Gas6-Protein und/oder Komplementfaktor B im Plasma von an einer schweren Sepsis oder einem septischen Schock leidenden Personen verwendet werden.

## Beschreibung

### GEBIET DER ERFINDUNG

Die vorliegende Erfindung bezieht sich auf ein extrakorporales Blut- oder Plasmäreinigungssystem und ebenso auf adsorbierende Partikel, die eine spezifische Adsorption von Blutplasmakomponenten ermöglichen, welche bedeutende Faktoren in der Pathogenese einer schweren Sepsis und eines septischen Schocks sind, sowie auf die Verwendung davon.

### HINTERGRUND DER ERFINDUNG

Der Begriff "Sepsis" beschreibt ein Spektrum klinischer Symptome, die durch eine heftige und fehlgesteuerte systemische Immunreaktion des Körpers auf eine Infektion verursacht werden. Eine Diagnose ist in klinischer Hinsicht schwierig, und die Behandlungsmöglichkeiten sind eingeschränkt. Der klinische Ausbruch kann Fieber oder Hypothermie, geistige Verwirrung, Tachykardie, eine vorübergehende Hypotonie, eine verminderte Urinausscheidung und eine unerklärliche Thrombozytenverminderung, eine erhöhte oder verringerte Leukozytenzahl und eine rasche Atmung umfassen (siehe Annane et al. 2005). Anschließend können sich Gerinnungsanomalien und eine schwere Hypotonie entwickeln, was zu einem Organversagen führt (Cohen 2002, Hotchkiss und Karl 2003).

In der internationalen Definition kommt es zu einer schweren Sepsis, wenn eine Sepsis in Verbindung mit Problemen bei einem oder mehreren der lebenswichtigen Organe auftritt. Ein septischer Schock tritt auf, wenn eine schwere Sepsis durch einen niedrigen Blutdruck, der nicht auf eine Standardbehandlung anspricht, erschwert wird (Annane et al. 2005). Die Sterblichkeitsrate beträgt 30-50%, abhängig von der Schwere der Sepsis und trotz Behandlung in einer Intensivstation (ICU). Die Häufigkeit des Auftretens wird auf 750.000 Patienten mit jährlich 215.000 Todesfällen geschätzt (Angus et al. 2001), wodurch dies zur zehnthäufigsten Todesursache in den Vereinigten Staaten wird. (Hoyert et al. 1999). Eine jüngste Analyse der Epidemiologie der Sepsis in den Vereinigten Staaten zeigte, dass das Auftreten von Sepsis und die Anzahl der mit Sepsis zusammenhängenden Todesfälle zunimmt (Martin et al. 2003). Innerhalb der europäischen Union sterben jedes Jahr mehr als 100.000 Personen an schwerer Sepsis und septischem Schock (Davies et al. 2001). Somit ist die Sepsis ein großes Problem der Medizin und Gesundheitsvorsorge.

Bei einer Sepsis kann in etwa der Hälfte der Fälle eine mikrobiologische Diagnose erstellt werden; auf gramnegative Bakterien entfallen 30-40% der Fälle und auf grampositive etwa 50%, wobei es sich beim Rest um polymikrobielle Infektionen und Pilzinfektionen handelt (Alberti et al. 2002, Angus et al. 2001, Annane et al. 2005, Martin et al. 2003). Bei der gramnegativen Sepsis sind Infektionen exogenen Ursprungs, wie z.B. eine Meningokokkensepsis, in der Minderzahl. Endogene Infektionen, die von Organismen verursacht werden, welche von der körpereigenen Flora des Patienten stammen, wie z.B. *E. coli* und Mitglieder der *Enterobacteriaceae-*Familie, machen die meisten Fälle einer gramnegativen Sepsis aus (Papasian und Morrison 2002). Außerdem kann auch bei einer grampositiven Bakteriämie häufig eine Endotoxämie nachgewiesen werden (Annane et al. 2005).

Es ist allgemein anerkannt, dass die bakteriellen Endotoxine in der Blutbahn Monozyten aktivieren, indem sie sich an einen Toll-artigen Rezeptor des Typs 4 binden und somit Signalübermittlungskaskaden einleiten, welche zu Gentranscription und Proteintranslation und zur Absonderung zahlreicher Entzündungsvermittler führen (z.B. Cohen 2002). Zu den wichtigsten abgesonderten proinflammatorischen Zytokinmediatoren zählen TNF-α und IL-1β. Diese Zytokine wirken auf verschiedene Zielzellen, einschließlich Endothelzellen und Neutrophile. Als Ergebnis werden sowohl proinflammatorische als auch prokoagulante Mechanismen erheblich verstärkt. Endothelzellen steigern z.B. die Expression verschiedener Adhäsionsmoleküle und chemisch anziehender Moleküle. Die aktivierten Neutrophile, die von der Oberfläche des Endothels angezogen werden, setzen Proteasen und proinflammatorische Mediatoren frei und fördern somit eine weitere Gefäßverletzung.

Überreichlicher Gewebefaktor (Gerinnungsfaktor III), der von verschiedenen Zellquellen, einschließlich extravaskulärer Quellen, stammt, wird bei Fehlen einer ausreichenden Menge an aktiviertem Protein C in Thrombin umgewandelt, wodurch Blutgerinnungskaskaden eingeleitet werden. Protein C wird infolge einer verminderten Thrombomodulin-Expression in den Endothelzellen aufgrund der Wirkungen von TNF-α abgereichert. Die natürlichen Antikoagulationsmechanismen werden gestört, und die normalen fibrinolytischen Mechanismen werden durch das Vorhandensein hoher Spiegel von Plasminogenaktivatorinhibitoren (PAI-1) gestört. Außerdem wird das Komplement aktiviert.

Die proinflammatorischen und prokoagulanten Mechanismen tragen bekanntermaßen zur Gewebeischämie, zu Mikrothrombosen und zu einer verringerten Sauerstoffsättigung des Gewebes bei, was zum Organversagen führt. Gleichzeitig oder als Reaktion auf die proinflammatorische Phase kommt es zu einer Hochregulierung/Aktivierung mehrerer entzündungshemmender Mechanismen (z.B. IL-10), die in einer bestimmten Stufe der Erkrankung vorherrschen können (Hotchkiss und Karl 2003). Einige Forscher nehmen an, dass eine (Endokrin-vermittelte) metabolische Hinunterregulierung und eine verringerte Mitochondrienatmung zum fortschreitenden Organversagen beitragen (Annane et al. 2005, Cohen 2002, Hotchkiss und Karl 2003, Singer et al. 2004).

Trotz eines besseren Verständnisses der pathologischen Physiologie der Sepsis sind in den vergangenen Jahren zahlreiche klinische Versuche hinsichtlich neuer pharmazeutischer Produkte fehlgeschlagen. Beispielsweise verringerten monoklonale anti-TNF-α-Antikörper, einschließlich muriner anti-TNF (Cohen et al. 1996, Abraham et al. 1998), muriner anti-TNF-α F(ab')₂-Fragmente (Reinhart et al. 1996, Natanson et al. 1998) und humanisierter chimärer anti-TNF (Clark et al. 1998), TNF-α-Rezeptorfusionsproteine (Fisher et al. 1996) und IL-1ra (Fisher et al. 1994, Opal et al. 1997), nicht die Morbidität oder Mortalität von lebensgefährlich an Sepsis erkrankten Patienten. Gleichermaßen zeigten Studien, bei denen unter Verwendung von Antithrombin III (ATIII) (Warren et al. 2001) oder eines Tissue-Factor-Pathway-Inhibitors (TFPI) (Abraham et al. 2003) auf das Gerinnungssystem abgezielt wurde, keinen signifikanten Vorteil.

Wie obenstehend erwähnt, ist das aktivierte Protein C (rAPC), die rekombinante Form eines natürlichen Antikoagulans, bisher das einzige neue pharmazeutische Produkt, das in einem kontrollierten klinischen Phase III-Versuch einen signifikanten Überlebensvorteil aufwies. Die absolute Verringerung der 28-Tage-Sterblichkeitsrate betrug in der PROWESS-Studie 6,1% (30,8% Placebo-Gruppe; 24,7% rAPC-Gruppe) (Bernard et al. 2001). Einer der Nachteile besteht darin, dass es zu einem gesteigerten Blutungsrisiko kommt, wie in der PROWESS-Studie beschrieben. In anderen klinischen Studien zeigte sich, dass eine aggressive Handhabung einer hämodynamischen Homöostase (Rivers et al. 2001), eine intensive Insulintherapie (Van den Berghe et al. 2001) und Kortikosteroide in geringer Dosierung (Annane et al. 2002) bei einer Sepsis von Vorteil sind. Zusammengenommen zeigen diese Daten, dass die Behandlungsoptionen bei lebensgefährlich an Sepsis erkrankten Patienten nach wie vor eingeschränkt sind.

### Extrakorporale Blutreinigung unter Anwendung einer spezifischen Adsorption im Vergleich zum pharmazeutischen Therapieansatz

Extrakorporale Blutreinigungssysteme beruhen in der klinischen Anwendung derzeit auf der Trennung von Blut in eine Zell- und eine Plasmafraktion entweder mittels Zentrifugation oder mittels Hohlfaserfilter in einem Kreislauf außerhalb des Körpers des Patienten. Pathogene Faktoren werden durch das Binden an funktionalisierte Polymerträger, die in Adsorptionssäulen zurückgehalten werden, aus der Plasmafraktion entfernt, und das gereinigte Plasma wird anschließend zusammen mit den Blutzellen dem Patienten wieder zugeführt.

Ein Hauptvorteil der spezifischen Adsorption besteht darin, dass nur das Zielmolekül bzw. die Zielmoleküle adsorbiert wird bzw. werden und alle anderen Moleküle, die nicht entfernt werden sollten, im Plasma verbleiben, wodurch nachteilige Wirkungen der Therapie minimal gehalten werden. Das auf Mikrokügelchen basierende Entgiftungssystem ("Microspheres-Based Detoxification System": MDS; D. Falkenhagen, H. Schima, F. Loth: Arrangement for Removing Substances from Liquids, in particluar Blood. Europäisches Patent: 94 926 169.7; japanisches Patent Nr. 501083/97; U.S.-Patent: 5,855,782 / 5. Jänner 1999; PCT EP/94/02644 (WO 95/04559)) ist ein zu herkömmlichen Blutreinigungssystemen alternativer Ansatz (Weber et al. 1994, Weber und Falkenhagen 1996). In diesem Fall durchtränkt das Plasma nicht eine Adsorptionssäule, sondern wird zusammen mit spezifischen adsorbierenden Mikropartikeln in der Filtratkammer des Moduls zirkulieren gelassen. Die spezifischen adsorbierenden Mikropartikel (1-10 µm im Durchmesser) ermöglichen die rasche Entfernung von Zielsubstanzen. Die Hauptvorteile des MDS umfassen (1) eine hohe Adsorptionsfähigkeit aufgrund des hohen Oberfläche-Volumen-Verhältnisses der Partikel; (2) eine ausgezeichnete Adsorptionskinetik aufgrund geringer Diffusionsdistanzen und (3) eine hohe Flexibilität, da das System für die Adsorption verschiedener Faktoren verwendet werden kann, abhängig von der Spezifität der eingesetzten Adsorptionsmittel.

Da die meisten pathophysiologischen Mediatoren in einer Sepsis von ihrer Natur her pleiotrop sind, ist es denkbar, dass ein Absenken der Konzentration eines bestimmten Faktors im Blut bei der Therapie von Sepsis-Patienten günstig ist ("Peak-Cutting"). Eine vollständige Beseitigung oder Blockierung des Zielmoleküls in den Körpergeweben ist jedoch nicht wünschenswert. TNF-α und IL-6 sind Beispiele für proinflammatorische Mediatoren, die für ein zelluläres Signalisieren unter bestimmten Bedingungen (Abheilung, Rückgang einer Entzündung) notwendig sein könnten und daher nicht vollständig entfernt werden sollten (Arnett et al. 2003, Wüstefeld et al. 2003). Diesbezüglich ist es äußerst schwierig, eine systemische Antikörpertherapie (d.h. einen als Arzneimittel zugeführten Antikörper) zu steuern, die für den Patienten in der Tat schädlich sein kann, wenn die Konzentration des Zielmoleküls in der Blutbahn und lokal in Geweben zu gering wird (d.h. innerhalb des Verteilungsvolumens des Arzneimittels).

Bei einer extrakorporalen Blutreinigungstherapie kann die Blutkonzentration eines bestimmten Zielfaktors durch eine spezifische Adsorption genauer gesteuert werden, da die Therapie sofort eingestellt werden kann. Außerdem erschöpft eine extrakorporale Blutreinigung nicht die lokalen Gewebespiegel des Zielfaktors, welcher adsorbiert wird, und schafft somit einen Vorteil gegenüber einer systemischen Arzneimittelverabreichung.

Wie obenstehend erwähnt, spielen Blutgerinnung und Zelladhäsion bei der Pathogenese einer Sepsis eine bedeutende Rolle. Es ist bekannt, dass das Freisetzen von Gewebefaktor (Faktor III) infolge einer Gefäßverletzung den extrinsischen Weg einer Sepsis einleitet, welche dann durch den intrinsischen Weg verstärkt wird (Cohen 2002, Price et al. 2004). Außerdem spielen mehrere Adhäsionsmoleküle, z.B. lösliche L-, P- und E-Selektine, das interzelluläre Adhäsionsmolekül-1 (sICAM-1), das vaskuläre Zelladhäsionsmolekül-1 (sVCAM-1), infolge von proinflammatorischen Reizen bei der pathologischen Physiologie eine Rolle. Es zeigte sich, dass zum Beispiel eine ICAM-Expression in menschlichen Koronarendothelzellen durch aktiviertes Protein C reduziert wird (Franscini et al. 2004). Folglich wäre bei der Behandlung von Sepsis-Patienten eine Verringerung der Gerinnung und Adhäsion vorteilhaft.

### Gas6/Tyro3-Signalisierung

Tyro3 (auch bekannt als Sky, Rse, Dtk, Etk, Brt und Tif) gehört zu Rezeptortyrosinkinasen der Axl/Tyro3-Unterfamilie. Der Rezeptor wird in verschiedenen Gewebe-/Zelltypen von Erwachsenen, einschließlich des Gefäßsystems, Lymphsystems, Gehirns und der Hoden, häufig zumindest in einem geringen Maß exprimiert (Crosier und Crosier 1997, Godowski et al. 1995, Nagata et al. 1996). Bei der neuronalen Entwicklung (Lai et al. 1994) und bei der Immunregulierung (Lu und Lemke 2001) besitzt er bekanntermaßen eine wichtige Funktion. In seiner Plasmamembran-gebundenen Form enthält der vorhergesagte extrazelluläre N-Terminus zwei Immunglobulin-artige Bereiche (Ig-Bereiche), gefolgt von zwei Fibronektin-Typ III-Wiederholungen. Nach dem Transmembranbereich folgt ein zytoplasmatischer Tyrosinkinasebereich im C-Terminus des Proteins.

Es gibt Hinweise darauf, dass der Tyro3-Rezeptor an der Zelladhäsion beteiligt ist (Bellosta et al. 1995). Strukturelle Studien haben gezeigt, dass die extrazellulären Bereiche der Tyro3-Rezeptoren an der Bildung von Homodimeren beteiligt sind, und zwar sogar bei Fehlen von deren Gas6-Liganden (Heiring et al. 2004). Interessanterweise wird auf Basis einer bioinformatischen Analyse hinsichtlich der Signalsequenz vorausgesagt, dass Tyro3 im extrazellulären Raum, einschließlich des Blutes, auch in sekretierter löslicher Form zu finden ist. Zusammenfassend ist zu sagen, dass mehrere Beweislinien darauf hindeuten, dass der Tyro3-Rezeptor zusätzlich zu seinen signalübermittelnden Funktionen durch die Rezeptorkinase mit der Adhäsion zusammenhängende Funktionen aufweist.

Gas6, das Produkt des für die Wachstumshemmung spezifischen Gens 6, wurde als bisher einziger Ligand für den Tyro3-Rezeptor identifiziert. Gas6 besitzt die höchste Sequenzhomologie (46%) zum Protein S, einem Kofaktor des aktivierten Proteins C, welche beim Abbau der prokoagulanten, aktivierten Faktoren V und VIII zusammenwirken (DiScipio et al. 1979, Walker et al. 1980, 1987, Manfioletti et al. 1993). Ähnlich wie das Protein S, das Protein C und die Gerinnungsfaktoren II (Prothrombin), VII (Prokonvertin), IX (Christmas-Faktor, Plasma-Thromboplastin-Komponente) und X (Stuart-Faktor) ist Gas6 ein Vitamin K-abhängiges Protein (Manfioletti et al. 1993). All diese Vitamin K-abhängigen Proteine enthalten die Aminosäure γ-Carboxyglutaminsäure (Gla) und alle nehmen an Reaktionen, die Calcium erfordern, teil. Vitamin K ist beteiligt an der Umwandlung von 10-12 Glutaminsäureresten in Vorläuferproteinen (z.B. Prothrombin-Voräufer) zu ihren aktiven Formen (z.B. Prothrombin) durch Hinzufügen von Kohlendioxid (Carboxylierung). Dieses Hinzufügen erhöht die Affinität von Glutaminsäureresten hinsichtlich Calcium.

Es wurde nachgewiesen, dass der Gla-Bereich von Gas6 die Verbindung des Proteins mit biologischen Membranen ermöglicht (Nakano et al. 1997). Gas6 ist in normalem Plasma in einem geringen Ausmaß (0,16-0,28 nM) vorhanden, und es wurde berichtet, dass die Konzentration bei Patienten, die das orale gerinnungshemmende Warfarin erhielten, geringer war (Balogh et al. 2005).

Die Bindungsaffinität von Gas6 an den Tyro3-Rezeptor liegt im nanomolaren Bereich (2,9 nM) (Nagata et al. 1996). Die Bindung von Gas6 an den Tyro3-Rezeptor leitet eine Phosphorylierung durch die Rezeptortyrosinkinase und eine anschließende Signalübermittlung ein. Es zeigte sich, dass die Tyro3-Rezeptor-Aktivierung die Knochenresorption in Osteoklasten durch MAPK-Mechanismen (Katagiri et al. 2001) und die endokrine Differenzierung in Trophoblasten durch PI3-K/akt-Mechanismen (Kamei et al. 2002) aktiviert. Bisher sind die Informationen der Signalübermittlungsmechanismen des Gas6-Liganden/Tyro3-Rezeptors in anderen Zelltypen beschränkt.

Es wurde nachgewiesen, dass Gas6 die Blutplättchenaggregation und -sekretion verstärkt und die Bildung stabiler Blutplättchen-Makroaggregate als Reaktion auf bekannte Agonisten (z.B. Thrombin) fördert. Außerdem wurde an Knock-Out-Mäusen nachgewiesen, dass eine Inaktivierung des Gens bei Mäusen eine Venen- und Arterienthrombose verhinderte und die Tiere vor einer tödlichen Kollagen/Epinephrin-induzierten Thromboembolie ohne spontane Blutung schützte (Angelillo-Scherrer et al. 2001). In einer nachfolgenden Studie schützte das Ausschalten des Tyro3-Rezeptors bei Mäusen die Tiere vor einer lebensbedrohlichen Thrombose und beeinträchtigte außerdem die Stabilisierung von Blutplättchenaggregaten (Angelillo-Scherrer et al. 2005).

Interessanterweise wurde berichtet, dass Gas6 auch die Expression des Gewebefaktors (Gerinnungsfaktor III) in Endothelzellen reguliert. Angelillo-Scherrer et al. (2005) kamen zu dem Schluss, dass Gas6, das durch seine Rezeptoren (z.B. Tyro3) signalisiert, zu einer Klasse von Molekülen gehört, die bei einer Grundlinien-Hämostase redundant sind, aber in pathologischen Zuständen ein wichtiges Amplifikationssystem bilden. Zusammenfassend ist zu sagen, dass Gas6/Tyro3 bei der prokoagulanten Aktivität eine wichtige biologische Funktion besitzen.

### Komplementfaktor B

Mehrere Beweislinien weisen auf die Rolle des Komplements bei einer Sepsis hin (Ward 2004, Gerard, 2003). Das Komplementsystem ist mit dem Gerinnungssystem und dem fibrinolytischen System funktionell verknüpft. In der Literatur finden sich fundierte Daten, die das Erscheinungsbild von Komplementaktivierungsprodukten, insbesondere C3a und C5a, im Plasma von Sepsis-Patienten darstellen. Ein beständiges Ansteigen dieser Anaphylatoxine scheint mit der Entwicklung eines Multiorganversagens zusammenzuhängen und steht mit dem Überleben in einer umgekehrten Beziehung (Nakae et al., 1994). Hohe zirkulierende Spiegel von C5a beeinflussen Neutrophile (Lähmung der neutrophilen Signalisierungswege, reduzierte Bildung von H₂O₂, stark verringerte phagozytische Fähigkeit), Phagozyten (gesteigerte Produktion von proinflammatorischen Mediatoren wie z.B. TNF-α) sowie Endothelzellen (gesteigerte Produktion von proinflammatorischen Mediatoren, Bildung von Gewebefaktor).

Es zeigte sich, dass eine Anti-C5a-Behandlung in Tiermodellen hinsichtlich einer Sepsis die Entwicklung einer fehlerhaften Neutrophilenfunktion und eines Multiorganversagens verhindert (Huber-Lang et al. 2001). Obwohl alle drei Wege, der klassische Weg, der Lektinweg und der alternative Weg, an einer polymikrobiellen Sepsis beteiligt sein können (z.B. Celik et al., 2001), gibt es einen wesentlichen Hinweis darauf, dass der alternative Komplementweg bei der Endotoxin-induzierten Sepsis eine wichtige Rolle spielt (Clardy 1994). Der alternative Weg erfüllt wichtige Funktionen beim Verstärken einer Opsonisierung und Chemotaxis (Walport, 2001).

### Die drei Wege des Komplementsystems (von Walport 2001)

Kurz gesagt, der alternative Weg wird nach dem Binden des Faktors B an C3b zur Bildung von C3bB aktiviert, welches anschließend durch den Faktor D gespalten wird, um den Alternativwegkomplex C3bBb zu bilden. Der durch Properdin stabilisierte Monomerkomplex dient als C3/C5-Konvertase von geringer Affinität, welche in erster Linie C3-Moleküle zur Bildung neuer C3b-Moleküle spalten kann. Eine erhöhte Ablagerung von C3b zur Bildung von [C3bBb(C3b)ₙ] an der Oberfläche des Komplementaktivators (z.B. Bakterienmaterial) begünstigt die Anhaftung von C5 mit höherer Affinität, wobei die C3/C5-Konvertase von geringer Affinität in eine C5-Konvertase von hoher Affinität umgewandelt wird (Rawal und Pangburn, 2001). Dieses Enzym spaltet C5, um das Anaphylatoxin C5a und C5b zu bilden, welches die Membranangriffskomplexbildung einleitet. Somit spielt der alternative Weg bei der Amplifikation eine wichtige Rolle.

Normalerweise sind speziesspezifische Membraninhibitoren für Komplement vorhanden, wodurch die Wirtszelloberflächen vor dem homologen Komplement geschützt werden (Okada et al., 1983; Lachmann 1991). Eine Studie von Brodbeck et al. (2000) zeigte, dass eine konzentrationsabhängige kompetitive Beziehung zwischen Faktor B (und D), welche das Zusammenfügen des C3bBb-Komplexes fördern, und den Komplementregulatoren CD46 (Membran-Cofaktor-Protein, MCP) und CD55 (zerfallsbeschleunigender Faktor, DAF), welche eine Wirtszellen-gebundene C3b-Spaltung zu inaktiviertem C3b durch den Faktor I fördern, besteht.

Diese Daten weisen auf die Wichtigkeit der Konzentration des Faktors B in Bezug auf Komplementregulatoren hin. Eine gestörte Homöostase zwischen Aktivatoren und Inhibitoren des Komplements an Wirtszelloberflächen könnte potentiell zu einer dramatischen Beschädigung des Wirts führen. In der Tat wurde nahegelegt, dass die Bildung von C3-Konvertasen (C3bBb), die sich an Wirtszellmembranen absetzen, an einem Endotoxinschock beteiligt sein könnte (z.B. Mizuno et al., 1999). Weiters wurde gezeigt, dass ein Endothelschaden mittels LPS durch Zytokine hervorgerufen werden kann, was zur Freilegung der subendothelialen extrazellulären Matrix (ECM) führt. Die ECM-Freilegung kann potentiell eine Ablagerung von Komplementfaktoren bewirken und somit zu einem Gefäßschaden bei einer Sepsis und einem Multiorganversagen beitragen (Hindmarsh und Marks, 1998).

Es wird angenommen, dass der Komplementfaktor B des alternativen Weges hauptsächlich von der Leber synthetisiert und sekretiert wird (Alper et al. 1980; Morris et al., 1982). Es zeigte sich jedoch, dass er auch von anderen Zelltypen, wie z.B. Monozyten/Phagozyten (Laszlo et al., 1993), Fibroblasten (Katz et al., 1988) und Endothelzellen (Ripoche et al., 1988), synthetisiert und sekretiert wird. Die nicht zur Leber gehörigen Stellen, d.h. insbesondere das Endothel besitzt aufgrund seiner äußerst großen Oberfläche das Potential, erheblich zur Faktor B-Sekretion beizutragen. Frühere Berichte zeigten bereits, dass TNF-α allein sowie synergistisch mit IFN-γ (Kawakami et al., 1997; Vastag et al., 1998) und IL-1α (Coulpier et al., 1995) die Sekretion von Faktor B in menschlichen Nabelschnurendothelzellen (HUVECs) und menschlichen Gehirnendothelzellen, die aus Mikrogefäßen isoliert wurden, erhöhte.

Interessanterweise wies eine jüngste Studie nach, dass die Promotorstelle des Faktor B-Gens eine NF-κB *cis*-Bindungsstelle enthält (Huang et al., 2002). Eine Mutation des NF-κB-Elements schwächte die Wirkung von TNF-α ab und verminderte die synergistischen Wirkungen von TNF-α und IFN-γ auf die Faktor B-Genexpression in murinen Phagozyten. Zusammenfassend weisen diese Daten darauf hin, dass eine Faktor B-Genexpression durch einen NF-κB-vermittelten Mechanismus und somit direkt durch TNF-α geregelt wird, und zwar auch in menschlichen Endothelzellen.

Daten aus unserem eigenen Labor, in dem wir unter Verwendung von HUVEC ein experimentelles Sepsis-Modell errichteten, unterstützen diese Erkenntnisse. Nach der Stimulierung von menschlichen monozytären THP-1-Zellen mit LPS (oder einem Vehikel als Kontrolle) in Gegenwart von menschlichem Plasma der Gruppe AB wurde das Medium gesammelt. Anschließend wurden HUVECs dem geernteten Medium (ohne THP-1-Zellen) ausgesetzt, und das Expressionsprofil von menschlichen 10K-Genen wurde sowohl bei THP-1-Zellen als auch bei HUVECs in drei unabhängigen Mikroarray-Versuchen untersucht. In diesen Versuchen wurden mehrere bekannte Faktoren durch LPS (z.B. TNF-α, IL-1β, IL-8 in THP-1-Zellen und IL-1α, IL-6, IL-8, TLR-2, SOD2 in HUVECs) hochreguliert, wodurch die Zuversicht geschaffen wurde, dass die Versuchsbedingungen gültig sind. Unter den hochregulierten Genen wurde die mRNA für Tyro3 verglichen mit der Kontrolle in allen drei Versuchen in THP-1-Zellen und HUVECs beständig induziert. Die mRNA für Faktor B wurde verglichen mit der Kontrolle in allen drei Versuchen in HUVECs beständig induziert.

Es wird angenommen, dass unter den Bedingungen einer gramnegativen Sepsis LPS zusammen mit TNF-α und anderen sekretierten Zytokinen und Faktoren in der Blutbahn eine systemische Hochregulierung der Tyro3/Gas6-Signalisierung und Komplementfaktor B-Aktivität bewirken kann. Eine Entfernung von überschüssigem löslichem Tyro3/Gas6 ist bei der Verringerung der prokoagulanten Aktivität möglicherweise von Vorteil. Eine Absenkung der Konzentration an Komplementfaktor B, jedoch nicht eine vollständige Neutralisierung, kann für die Patienten ebenfalls vorteilhaft sein, wie obenstehend beschrieben. Der letztere Ansatz lässt den klassischen Weg und den Mannose-bindenden Lektinweg des Komplements intakt, was für die Verteidigung des Wirts entscheidend ist.

### OFFENBARUNG DER ERFINDUNG

Eine Aufgabe der vorliegenden Erfindung besteht daher in der Bereitstellung eines Systems, das eine Verringerung der obenstehend erwähnten wichtigen Faktoren bei der Pathogenese einer schweren Sepsis und eines septischen Schocks, nämlich von Tyro3-Rezeptorprotein, Gas6-Protein und Komplementfaktor B, im Blutplasma ermöglicht.

Diese Aufgabe wird durch ein extrakorporales Blut- oder Plasmareinigungssystem erfüllt, umfassend ein für das Tyro3-Rezeptorprotein spezifisches Adsorptionsmittel und/oder ein für das Gas6-Protein spezifisches Adsorptionsmittel und/oder ein für Komplementfaktor B spezifisches Adsorptionsmittel.

Eine bevorzugte Ausführungsform des Reinigungssystems ist dadurch gekennzeichnet, dass das für das Tyro3-Rezeptorprotein spezifische Adsorptionsmittel eine Funktionalität, ausgewählt aus der Gruppe, umfassend einen polyklonalen Antikörper gegen Tyro3, einen monoklonalen Antikörper gegen Tyro3, ein monoklonales Antikörperfragment gegen Tyro3 und ein spezifisch an Tyro3 bindendes Peptid, umfasst.

Eine weitere bevorzugte Ausführungsform ist dadurch gekennzeichnet, dass das für das Gas6-Protein spezifische Adsorptionsmittel eine Funktionalität, ausgewählt aus der Gruppe, umfassend einen polyklonalen Antikörper gegen Gas6, einen monoklonalen Antikörper gegen Gas6, ein monoklonales Antikörperfragment gegen Gas6 und ein spezifisch an Gas6 bindendes Peptid, umfasst.

Eine weitere bevorzugte Ausführungsform ist dadurch gekennzeichnet, dass das für den Komplementfaktor B spezifische Adsorptionsmittel eine Funktionalität, ausgewählt aus der Gruppe, umfassend einen polyklonalen Antikörper gegen Komplementfaktor B, einen monoklonalen Antikörper gegen Komplementfaktor B, ein monoklonales Antikörperfragment gegen Komplementfaktor B und ein spezifisch an Komplementfaktor B bindendes Peptid, umfasst.

Gemäß einer weiteren Ausführungsform wird das Adsorptionsmittel auf einem festen Träger, vorzugsweise auf wasserunlöslichen Trägerpartikeln, immobilisiert.

Geeigneterweise werden die Trägerpartikel zur Immobilisierung des Adsorptionsmittels vorzugsweise mit Natriummetaperiodat, Epichlorhydrin oder Butandioldiglycidylether aktiviert.

Vorzugsweise sind die Trägerpartikel Cellulose-Mikrokügelchen.

Gemäß einem weiteren Aspekt der Erfindung wird das erfinderische extrakorporale Blut- oder Plasmareinigungssystem zum Absenken der Konzentration an Tyro3-Rezeptorprotein und/oder Gas6-Protein und/oder Komplementfaktor B im Plasma von an einer schweren Sepsis oder einem septischen Schock leidenden Personen verwendet.

Die Erfindung stellt weiters adsorbierende Partikel zur Verwendung in einem extrakorporalen Blut- oder Plasmareinigungssystem bereit, umfassend ein für das Tyro3-Rezeptorprotein spezifisches Adsorptionsmittel und/oder ein für das Gas6-Protein spezifisches Adsorptionsmittel und/oder ein für Komplementfaktor B spezifisches Adsorptionsmittel.

Vorzugsweise umfasst das Adsorptionsmittel eine Funktionalität, ausgewählt aus der Gruppe, umfassend einen polyklonalen Antikörper, einen monoklonalen Antikörper, ein monoklonales Antikörperfragment gegen Tyro3, Gas6 oder Komplementfaktor B und ein spezifisch an Tyro3, Gas6 oder Komplementfaktor B bindendes Peptid.

### Modulation (Absenkung) der Tyro3- und/oder Gas6- und/oder Komplementfaktor B-Konzentration im Plasma unter Verwendung einer spezifischen Adsorption

In unseren Studien entwickelten wir eine einzigartige, auf einer Zellkultur basierende Versuchsanordnung zur Untersuchung der pathophysiologischen Mechanismen einer gramnegativen Sepsis. In diesen Zellkulturstudien konzentrierten wir uns auf die Funktion der Monozyten und Endothelzellen bei der Endotoxin-induzierten Aktivierung. Wir optimierten die Zellkulturbedingungen, so dass diese *in vivo*-Bedingungen simulierten. Dies wird durch die Tatsache bewiesen, dass mehrere wohlbekannte Kennzeichen einer Sepsis im Zellkultursystem nachgewiesen wurden. In diesen Studien haben wir festgestellt, dass die Transcription von Tyro3-Rezeptorkinase nach dem Aussetzen an Endotoxin sowohl in monozytären THP-1- als auch in menschlichen Endothelzellen gesteigert wird. Gleichermaßen wurde mRNA für den Faktor B in menschlichen Endothelzellen induziert.

Da nachgewiesen wurde, dass der Rezeptor Tyro3 und sein Ligand Gas6 prokoagulante Eigenschaften und Adhäsionsmoleküleigenschaften (siehe obenstehende Literaturreferenzen) aufweisen, wird der Schluss gezogen, dass der Rezeptor bei der pathologischen Physiologie einer Endotoxin-induzierten Sepsis eine Rolle spielt. Somit besteht eine starke Veranlassung, die Plasmakonzentration von löslichem Tyro3 und/oder Gas6 zu modulieren (abzusenken), wobei bei einer extrakorporalen Blutreinigung beim Plasma von Patienten, die an einer Endotoxin-induzierten schweren Sepsis oder an einem Endotoxin-induzierten septischen Schock leiden, eine spezifische Adsorption angewandt wird.

Da der Komplementfaktor B ein Hauptfaktor des alternativen Weges ist, welcher an der Verstärkung der Komplementaktivität beteiligt ist, besteht gleichermaßen eine starke Veranlassung, die Plasmakonzentration an Komplementfaktor B unter ähnlichen Bedingungen zu modulieren (abzusenken).

### BEISPIELE

### Adsorptionsmittel

- Bildung eines spezifischen Adsorptionsmittels unter Verwendung eines polyklonalen Antikörpers gegen Tyro3 oder Gas6 oder Komplementfaktor B
- Bildung eines spezifischen Adsorptionsmittels unter Verwendung eines monoklonalen Antikörpers gegen Tyro3 oder Gas6 oder Komplementfaktor B
- Bildung eines spezifischen Adsorptionsmittels unter Verwendung eines monoklonalen Antikörperfragments gegen Tyro3 oder Gas6 oder Komplementfaktor B
- Bildung eines spezifischen Adsorptionsmittels unter Verwendung eines spezifisch an Tyro3 oder Gas6 oder Komplementfaktor B bindenden Peptids

### System

- Verwendung von spezifischen Adsorptionsmitteln in einer extrakorporalen Blutreinigungssituation
- Verfahren zur Anwendung einer spezifischen Adsorption von Gas6 und/oder Tyro3 und/oder Komplementfaktor B zur Behandlung von schwerer Sepsis und septischem Schock

Spezifische Adsorptionsmittel für Gas6 oder Tyro3 oder Komplementfaktor B können durch Funktionalisierung geeigneter wasserunlöslicher Trägerpartikel mit Liganden, wie z.B. poly- oder monoklonalen Antikörpern, rekombinanten menschlichen Antikörperfragmenten oder Peptiden, hergestellt werden. Eine Aktivierung der Partikel für die Immobilisierung funktioneller Liganden (Antikörper, Antikörperfragmente oder Peptide) kann in verschiedender Weise bewerkstelligt werden, z.B. mit (1) Natriummetaperiodat, (2) Epichlorhydrin oder (3) Butandioldiglycidylether.

Adsorptionsmittel sollten die folgenden Kriterien erfüllen:
1) spezifische Bindung der Zielmoleküle (Tyro3, Gas6, Komplementfaktor B)
2) ausreichende Bindungskapazität
3) chemische Stabilität, d.h. kein Austreten funktioneller Liganden
4) mechanische Stabilität
5) Biokompatibilität

### Beispiel 1

In einem Ausführungsbeispiel der Erfindung sind die Trägerpartikel Cellulose-Mikrokügelchen (Größenverteilung: 1 - 20 µm). Die Partikel werden mit Natriummetaperiodat aktiviert. Typische Aktivierungsgrade (Anteil an Dialdehydgruppen) liegen im Bereich von 300 bis 360 µmol pro Gramm Trockensubstanz. Poly- oder monoklonale Antikörper gegen Tyro3 und/oder Gas6 und/oder Komplementfaktor B werden über primäre Aminogruppen (d.h. in Zufallsanordnung) an den aktivierten Partikeln immobilisiert. Der Überschuss an aktivierten Gruppen wird mit einer Substanz gesättigt, die primäre Aminogruppen enthält, z.B. Ethanolamin.

### Beispiel 2

In einem weiteren Ausführungsbeispiel der Erfindung umfasst das extrakorporale Blut- oder Plasmareinigungssystem Trägerpartikel, wie in Beispiel 1 beschrieben, welche mit Epichlorhydrin und Iminodiessigsäure aktiviert werden (Weber et al. 2005). Rekombinante menschliche Antikörperfragmente, die eine C-terminale Hexahistidin-Markierung umfassen, werden an den aktivierten Cellulose-Mikrokügelchen immobilisiert. Aufgrund der Wechselwirkung der Histidin-Markierung mit den Chelatgruppen des Trägers werden die Antikörperfragmente mit der antigenbindenden Stelle nach außen ausgerichtet. Die rekombinanten menschlichen Fab-Fragmente gegen Gas6 oder Tyro3 oder Komplementfaktor B können unter Anwendung der Phagendarstellungstechnologie hergestellt werden.

Einzelne Kettenfragmente, die für Gas6 oder Tyro3 oder Komplementfaktor B spezifisch sind, werden aus Antikörperbibliotheken ausgewählt, und zwar durch eine Reihe von Verschiebungsrunden gegen rekombinantes Gas6 oder Tyro3 oder Komplementfaktor B unter Anwendung von Standardprotokollen. Einzelne Kettenfragmente werden in Fab-Fragmente umgewandelt, indem konstante menschliche Kappa- und menschliche IgG₁ CH1-Bereiche mit überlappender PCR kloniert werden. Zur gerichteten Kopplung der Fragmente wird in den C-Terminus der schweren Kette eine Hexahistidin-Markierung eingebracht.

### Beispiel 3

In einem weiteren Ausführungsbeispiel der Erfindung werden Trägerpartikel, wie in Beispiel 1 beschrieben, mit Natriummetaperiodat aktiviert. Typische Aktivierungsgrade (Anteil an Dialdehydgruppen) liegen im Bereich von 300 bis 360 µmol pro Gramm Trockensubstanz. Für Tyro3 oder Gas6 oder Komplementfaktor B spezifische Peptide werden kovalent an die Partikel gebunden. Für Gas6 oder Tyro3 oder Komplementfaktor B spezifische Peptide können unter Anwendung von Standardprotokollen aus Peptidbibliotheken isoliert werden.

Die Bestimmung der Affinität von Liganden und die Abschätzung der Adsorptionskapazität werden unter Anwendung von Standardverfahren durchgeführt.

Die erfindungsgemäßen spezifischen adsorbierenden Partikel können in einem extrakorporalen Blutreinigungssystem, wie z.B. dem auf Mikrokügelchen basierenden Entgiftungsystem, verwendet werden (MDS, D. Falkenhagen, H. Schima, F. Loth: Arrangement for Removing Substances from Liquids, in particluar Blood. Europäisches Patent: 94 926 169.7; Japanisches Patent Nr. 501083/97; U.S.-Patent: 5,855,782 / 5. Jänner 1999; PCT EP/94/02644 (WO 95/04559)) (Weber et al. 1994, Weber und Falkenhagen 1996).

### Angeführte Patentreferenzen

MDS: D. Falkenhagen, H. Schima, F. Loth: Arrangement for Removing Substances from Liquids, in particluar Blood. Europäisches Patent: 94 926 169.7; Japanisches Patent Nr. 501083/97; U.S.-Patent: 5,855,782 / 5. Jänner 1999; PCT EP/94/02644 (WO 95/04559))

### Literaturreferenzen:

Abraham E, Anzueto A, Gutierrez G, Tessler S, San Pedro G, Wunderink R, Dal Nogare A, Nasraway S, Berman S, Cooney R, Levy H, Baughman R, Rumbak M, Light RB, Poole L, Allred R, Constant J, Pennington J, Porter S. Double-blind randomised controlled trial of monoclonal antibody to human tumour necrosis factor in treatment of septic shock. NORASEPT II Study Group. Lancet. 351:929-33, 1998
Abraham E, Reinhart K, Opal S, Demeyer I, Doig C, Rodriguez AL, Beale R, Svoboda P, Laterre PF, Simon S, Light B, Spapen H, Stone J, Seibert A, Peckelsen C, De Deyne C, Postier R, Pettila V, Artigas A, Percell SR, Shu V, Zwingelstein C, Tobias J, Poole L, Stolzenbach JC, Creasey AA; OPTIMIST Trial Study Group. Efficacy and safety of tifacogin (recombinant tissue factor pathway inhibitor) in severe sepsis: a randomized controlled trial. JAMA 290(2):238-47, 2003
Alberti C, Brun-Buisson C, Burchardi H, Martin C, Goodman S, Artigas A, Sicignano A, Palazzo M, Moreno R, Boulme R, Lepage E, Le Gall R. Epidemiology of sepsis and infection in ICU patients from an international multicentre cohort study. Intensive Care Med. (2):108-21, 2002
Alper CA, Raum D, Awdeh ZL, Petersen PH, Taylor PD und Starzl TE: Studies of hepatic synthesis in vivo of plasma proteins, including orosomucoid, transferrin, α1-antitrypsin, C8, and factor B. Clin. Immunol. Immunopathol. 16: 84-89, 1980.
Angelillo-Scherrer A, de Frutos P, Aparicio C, Melis E, Savi P, Lupu F, Arnout J, Dewerchin M, Hoylaerts M, Herbert J, Collen D, Dahlback B, Carmeliet P. Deficiency or inhibition of Gas6 causes platelet dysfunction and protects mice against thrombosis. Nat Med. (2):215-21, 2001.
Angelillo-Scherrer A, Burnier L, Flores N, Savi P, DeMol M, Schaeffer P, Herbert JM, Lemke G, Goff SP, Matsushima GK, Earp HS, Vesin C, Hoylaerts MF, Plaisance S, Collen D, Conway EM, Wehrle-Haller B, Carmeliet P. Role of Gas6 receptors in platelet signaling during thrombus stabilization and implications for antithrombotic therapy. J Clin Invest. 115(2):237-46, 2005
Angus DC, Linde-Zwirble WT, Lidicker J, Clermont G, Carcillo J, Pinsky MR. Epidemiology of severe sepsis in the United States: analysis of incidence, outcome, and associated costs of care. Crit Care Med. 29(7):1303-10, 2001.
Annane D, Bellissant E, Cavaillon JM. Septic shock. Lancet. 365(9453):63-78, 2005.
Arnett HA, Wang Y, Matsushima GK, Suzuki K, Ting JP. Functional genomic analysis of remyelination reveals importance of inflammation in oligodendrocyte regeneration. J Neurosci. 23(30):9824-32, 2003.
Balogh I, Hafizi S, Stenhoff J, Hansson K, Dahlback B. Analysis of Gas6 in Human Platelets and Plasma. Arterioscler Thromb Vasc Biol: 24. März 2005.
Bellosta P, Costa M, Lin DA, Basilico C. The receptor tyrosine kinase ARK mediates cell aggregation by homophilic binding. Mol Cell Biol. 15(2):614-25, 1995.
Bernard GR, Vincent JL, Laterre PF, LaRosa SP, Dhainaut JF, Lopez-Rodriguez A, Steingrub JS, Garber GE, Helterbrand JD, Ely EW, Fisher CJ Jr; Recombinant human protein C Worldwide Evaluation in Severe Sepsis (PROWESS) study group. Efficacy and safety of recombinant human activated protein C for severe sepsis. N Engl J Med. 344(10):699-709, 2001.
Brodbeck WG, Mold C, Atkinson JP, and Medof ME: Cooperation between decayaccelerating factor and membrane cofactor protein in protecting cells from autologous complement attack. J. Immunol. 165: 3999-4006, 2000.
Clark MA, Plank LD, Connolly AB, Streat SJ, Hill AA, Gupta R, Monk DN, Shenkin A, Hill GL. Effect of a chimeric antibody to tumor necrosis factor-alpha on cytokine and physiologic responses in patients with severe sepsis--a randomized, clinical trial. Crit Care Med. 26(10):1650-9, 1998.
Celik I, Stover C, Botto M, Thiel S, Tzima S, Künkel D, Walport M, Lorenz W und Schwaeble W: Role of the classical pathway of complement activation in experimentally induced polymicrobial peritonitis. Infect. Immun. 69: 7304-7309, 2001.
INTERSEPT: an international, multicenter, placebo-controlled trial of monoclonal antibody to human tumor necrosis factor-alpha in patients with sepsis. International Sepsis Trial Study Group. Crit Care Med. 24(9):1431-40, 1996.
Clardy C: Complement activation by whole endotoxin is blocked by a monoclonal antibody to factor B. Infect. Immun. 62: 4549-4555, 1994.
Cohen J. The immunopathogenesis of sepsis. Nature 420(6917):885-91, 2002.
Coulpier M, Andreev S, Lemercier C, Dauchel H, Lees O, Fontaine M und Ripoche J: Activation of the endothelium by IL-1 alpha glucocorticoids results in major increase of complement C3 and factor B production and generation of C3a. Clin. Exp. Immunol. 101: 142-149, 1995.
Crosier KE, Crosier PS. New insights into the control of cell growth; the role of the AxI family. Pathology. 29(2):131-5, 1997.
Davies et al.: A European estimate of the burden of disease in ICU. European Society of Intensive Care Medicine Annual Meeting, Geneva, September 2001.
DiScipio RG, Davie EW. Characterization of protein S, a gamma-carboxyglutamic acid containing protein from bovine and human plasma. Biochemistry 8(5):899-904, 1979.
Fisher CJ Jr, Dhainaut JF, Opal SM, Pribble JP, Balk RA, Slotman GJ, Iberti TJ, Rackow EC, Shapiro MJ, Greenman RL, et al. Recombinant human interleukin 1 receptor antagonist in the treatment of patients with sepsis syndrome. Results from a randomized, double-blind, placebo-controlled trial. Phase III rhIL-1ra Sepsis Syndrome Study Group. JAMA 271 (23):1836-43, 1994.
Fisher CJ Jr, Agosti JM, Opal SM, Lowry SF, Balk RA, Sadoff JC, Abraham E, Schein RM, Benjamin E. Treatment of septic shock with the tumor necrosis factor receptor:Fc fusion protein. The Soluble TNF Receptor Sepsis Study Group. N Engl J Med. 334(26):1697-702, 1996.
Gerard C.: Complement C5a in the sepsis syndrome - too much of a good thing? N. Engl. J. Med. 348: 167-169, 2003.
Godowski PJ, Mark MR, Chen J, Sadick MD, Raab H, Hammonds RG. Reevaluation of the roles of protein S and Gas6 as ligands for the receptor tyrosine kinase Rse/Tyro 3. Cell. 11;82(3):355-8, 1995.
Heiring C, Dahlback B, Muller YA. Ligand recognition and homophilic interactions in Tyro3: structural insights into the Axl/Tyro3 receptor tyrosine kinase family. J Biol Chem. 20;279(8):6952-8, 2004.
Hindmarsh EJ und Marks RM: Complement activation occurs on subendothelial extracellular matrix in vitro and is initiated by retraction or removal of overlying endothelial cells. J. Immunol. 160: 67128-6136, 1998.
Hotchkiss RS, Karl IE. The pathophysiology and treatment of sepsis. N Engl J Med. 348(2):138-50, 2003.
Hoyert DL, Kochanek KD, Murphy SL. Deaths: final data for 1997. Natl Vital Stat Rep. 47(19):1-104, 1999.
Huang Y, Krein PM, Muruve DA und Winston BW: Complement factor B gene regulation: synergistic effects of TNF-α and IFN-γ in macrophages. J. Immunol. 169: 2627-2635, 2002.
Huber-Lang M, Sarma JV, McGuire S., Lu KT, Guo RF, Padgaonkar A, Younkin EM, Laudes IJ, Riedemann NC, Younger JG und Ward PA: Protectice effects of anti-C5a peptide antibodies in experimental sepsis. FASEB J. 15: 568-570, 2001.
Kamei T, Jones SR, Chapman BM, MCGonigle KL, Dai G, Soares MJ. The phosphatidylinositol 3-kinase/Akt signaling pathway modulates the endocrine differentiation of trophoblast cells. Mol Endocrinol. 16(7):1469-81, 2002.
Katagiri M, Hakeda Y, Chikazu D, Ogasawara T, Takato T, Kumegawa M, Nakamura K, Kawaguchi H. Mechanism of stimulation of osteoclastic bone resorption through Gas6/Tyro 3, a receptor tyrosine kinase signaling, in mouse osteoclasts. J Biol Chem. 276(10):7376-82, 2001.
Katz Y, Cole FS und Strunk RC: Synergism between γ. interferon and lipopolysaccharide for synthesis of factor B, but not C2, in human fibroblasts. J. Exp. Med. 167: 1-14, 1988.
Kawakami Y, Watanabe Y, Yamaguchi M, Sakaguchi K, Kono I und Ueki A.: TNF-alpha stimulates the biosynthesis of complement C3 and factor B by human umbilical cord vein endothelial cells. Cancer Lett. 116: 21-26, 1997.
Lachmann PJ: The control of homologous lysis. Immunol. Today 12: 312-315, 1991.
Lai C, Gore M, Lemke G. Structure, expression, and activity of Tyro 3, a neural adhesionrelated receptor tyrosine kinase. Oncogene. 9(9):2567-78, 1994.
Laszlo DJ, Henson PM, Weinstein L, Remigio LK, Sable C, Noble PW und Riches DWH: Development of functional diversity in mouse macrophages. Am. J. Pathol. 143: 587-597, 1993.
Lu Q, Lemke G. Homeostatic regulation of the immune system by receptor tyrosine kinases ofthe Tyro 3 family. Science 293(5528):306-11, 2001.
Manfioletti G, Brancolini C, Avanzi G, Schneider C. The protein encoded by a growth arrest-specific gene (gas6) is a new member of the vitamin K-dependent proteins related to protein S, a negative coregulator in the blood coagulation cascade. Mol Cell Biol. 13(8):4976-85, 1993.
Martin GS, Mannino DM, Eaton S, Moss M. The epidemiology of sepsis in the United States from 1979 through 2000. N Engl J Med. 348(16):1546-54, 2003.
Miller SI, Ernst RK, Bader MW. LPS, TLR4 and infectious disease diversity. Nat Rev Microbiol. 3(1):36-46, 2005.
Mizuno M, Nishikawa K, Okada N, Matsuo S, Ito K und Okada H: Inhibition of a membrane complement regulatory protein by a monoclonal antibody induces acute lethal shock in rats primed with lipopolysaccharide. J. Immunol. 162: 5477-5482, 1999.
Morris KM, Aden DP, Knowles PP und Colten HR: Complement biosynthesis by the human hepatoma-derived cell line HepG2. J. Clin. Invest. 70: 906-913, 1982.
Nagata K, Ohashi K, Nakano T, Arita H, Zong C, Hanafusa H, Mizuno K. Identification of the product of growth arrest-specific gene 6 as a common ligand for Axl, Sky, and Mer receptor tyrosine kinases. J Biol Chem. 271(47):30022-7, 1996.
Nakae H, Endo S, Inada K, Takakuwa T, Kasai T und Yoshida M: Serum complement levels and severity of sepsis. Res Commun Chem. Pathol. Pharmacol. 84: 189-195, 1994.
Nakano T, Kawamoto K, Kishino J, Nomura K, Higashino K, Arita H. Requirement of gamma-carboxyglutamic acid residues for the biological activity of Gas6: contribution of endogenous Gas6 to the proliferation of vascular smooth muscle cells. Biochem J.323 (Pt 2):387-92, 1997.
Okada H, Tanaka H und Okada N: Prevention of complement activation on the homologous cell membrane of nucleated cells as well as erythrocytes. Eur. J. Immunol. 13: 340-344, 1983.
Opal SM, Fisher CJ Jr, Dhainaut JF, Vincent JL, Brase R, Lowry SF, Sadoff JC, Slotman GJ, Levy H, Balk RA, Shelly MP, Pribble JP, LaBrecque JF, Lookabaugh J, Donovan H, Dubin H, Baughman R, Norman J, DeMaria E, Matzel K, Abraham E, Seneff M. Confirmatory interleukin-1 receptor antagonist trial in severe sepsis: a phase III, randomized, double-blind, placebo-controlled, multicenter trial. The Interleukin-1 Receptor Antagonist Sepsis Investigator Group. Crit Care Med. 25(7):1115-24, 1997.
Papasian C, Morrison DC. The pathogenesis of Gram-negative sepsis. In: The sepsis text. Hgb. JL Vincent, J. Carlet und SM. Opal. Kluwer Academic Publishers. S. 97-116, 2002.
Price GC, Thompson SA, Kam PC. Tissue factor and tissue factor pathway inhibitor. Anaesthesia 59(5):483-92, 2004.
Rawal N und Pangburn MK: Structure/function of C5 convertases of complement. Int. Immunopharmacol. 1: 415-422, 2001.
Reinhart K, Wiegand-Lohnert C, Grimminger F, Kaul M, Withington S, Treacher D, Eckart J, Willatts S, Bouza C, Krausch D, Stockenhuber F, Eiselstein J, Daum L, Kempeni J. Assessment of the safety and efficacy of the monoclonal anti-tumor necrosis factor antibodyfragment, MAK 195F, in patients with sepsis and septic shock: a multicenter, randomized, placebo-controlled, dose-ranging study. Crit Care Med. 24(5):733-42, 1996.
Ripoche J, Mitchell JA, Erdei A, Madin C, Moffatt B, Mokoena T, Gordon S und Sim RB: Interferon γ. induces synthesis of complement alternative pathway proteins by human endothelial cells in culture. J. Exp. Med. 168:1917-1922, 1988.
Rivers E, Nguyen B, Havstad S, Ressler J, Muzzin A, Knoblich B, Peterson E, Tomlanovich M; Early Goal-Directed Therapy Collaborative Group. Early goal-directed therapy in the treatment of severe sepsis and septic shock. N Engl J Med. 345(19):1368-77, 2001.
Singer M, De Santis V, Vitale D, Jeffcoate W. Multiorgan failure is an adaptive, endocrinemediated, metabolic response to overwhelming systemic inflammation. Lancet. 364(9433):545-8, 2004.
van den Berghe G, Wouters P, Weekers F, Verwaest C, Bruyninckx F, Schetz M, Vlasselaers D, Ferdinande P, Lauwers P, Bouillon R. Intensive insulin therapy in the critically ill patients. N Engl J Med. 345(19):1359-67, 2001.
Vastag M, Skopal J, Kramer J, Kolev K, Voko Z, Csonka E, Machovich R und Nagy Z: Endothelial cell cultured from human brain microvessels produce complement proteins factor H, factor B, C1 inhibitor, and C4. Immunobiology 199: 5-13, 1998.
Walker FJ. Regulation of activated protein C by a new protein. A possible function for bovine protein S. J Biol Chem. 255(12):5521-4, 1980.
Walport MJ: Complement. N. Engl. J. Med. 344: 1058-1066, 2001.
Ward P. (2004) The dark side of C5a in sepsis. Nature Rev. Immunol. 4: 133-142, 2004.
Warren BL, Eid A, Singer P, Pillay SS, Carl P, Novak I, Chalupa P, Atherstone A, Penzes I, Kubler A, Knaub S, Keinecke HO, Heinrichs H, Schindel F, Juers M, Bone RC, Opal SM; KyberSept Trial Study Group. Caring for the critically ill patient. High-dose antithrombin III in severe sepsis: a randomized controlled trial. JAMA. 286(15):1869-78, 2001.
Weber C, Falkenhagen D. Extracorporeal removal of proinflammatory cytokines by specific absorption onto microspheres. ASAIO J. 42(5):M908-11, 1996.
Weber C, Rajnoch C, Loth F, Schima H, Falkenhagen D. The Microspheres based Detoxification System (MDS). A new extracorporeal blood purification technology based on recirculated microspherical adsorbent particles. Int J Artif Organs. 17(11):595-602, 1994.
Weber, V, Linsberger I, Ettenauer M, Loth F, Höyhtyä M, Falkenhagen F: Develoment of specific adsorbents for human tumor nerosis factor-α: Influence of antibody immobolization on performance and biocompatibility. Biomacromolecules 6: 1864-1870, 2005.
Wuestefeld T, Klein C, Streetz KL, Betz U, Lauber J, Buer J, Manns MP, Muller W, Trautwein C. Interleukin-6/glycoprotein 130-dependent pathways are protective during liver regeneration. J Biol Chem. 278(13):11281-8, 2003.

## Patentansprüche

1. Extrakorporales Blut- oder Plasmareinigungssystem, umfassend ein für das Tyro3-Rezeptorprotein spezifisches Adsorptionsmittel und/oder ein für das Gas6-Protein spezifisches Adsorptionsmittel und/oder ein für Komplementfaktor B spezifisches Adsorptionsmittel.

2. System gemäß Anspruch 1, wobei das für das Tyro3-Rezeptorprotein spezifische Adsorptionsmittel eine Funktionalität, ausgewählt aus der Gruppe, umfassend einen polyklonalen Antikörper gegen Tyro3, einen monoklonalen Antikörper gegen Tyro3, ein monoklonales Antikörperfragment gegen Tyro3 und ein spezifisch an Tyro3 bindendes Peptid, umfasst.

3. System gemäß Anspruch 1 oder 2, wobei das für das Gas6-Protein spezifische Adsorptionsmittel eine Funktionalität, ausgewählt aus der Gruppe, umfassend einen polyklonalen Antikörper gegen Gas6, einen monoklonalen Antikörper gegen Gas6, ein monoklonales Antikörperfragment gegen Gas6 und ein spezifisch an Gas6 bindendes Peptid, umfasst.

4. System gemäß einem der Ansprüche 1 bis 3, wobei das für den Komplementfaktor B spezifische Adsorptionsmittel eine Funktionalität, ausgewählt aus der Gruppe, umfassend einen polyklonalen Antikörper gegen Komplementfaktor B, einen monoklonalen Antikörper gegen Komplementfaktor B, ein monoklonales Antikörperfragment gegen Komplementfaktor B und ein spezifisch an Komplementfaktor B bindendes Peptid, umfasst.

5. System gemäß einem der Ansprüche 1 bis 4, wobei das Adsorptionsmittel auf einem festen Träger, vorzugsweise auf wasserunlöslichen Trägerpartikeln, immobilisiert wird.

6. System gemäß Anspruch 5, wobei die Trägerpartikel zur Immobilisierung des Adsorptionsmittels, vorzugsweise mit Natriummetaperiodat, Epichlorhydrin oder Butandioldiglycidylether, aktiviert werden.

7. System gemäß Anspruch 5, wobei die Trägerpartikel Cellulose-Mikrokügelchen sind.

8. Verwendung des Systems gemäß einem der Ansprüche 1 bis 7 zum Absenken der Konzentration an Tyro3-Rezeptorprotein und/oder Gas6-Protein und/oder Komplementfaktor B im Plasma von an einer schweren Sepsis oder einem septischen Schock leidenden Personen.

9. Adsorbierende Partikel zur Verwendung in einem extrakorporalen Blut- oder Plasmareinigungssystem, umfassend ein für das Tyro3-Rezeptorprotein spezifisches Adsorptionsmittel und/oder ein für das Gas6-Protein spezifisches Adsorptionsmittel und/oder ein für Komplementfaktor B spezifisches Adsorptionsmittel.

10. Adsorbierende Partikel gemäß Anspruch 9, wobei das Adsorptionsmittel eine Funktionalität, ausgewählt aus der Gruppe, umfassend einen polyklonalen Antikörper, einen monoklonalen Antikörper, ein monoklonales Antikörperfragment gegen Tyro3, Gas6 oder Komplementfaktor B und ein spezifisch an Tyro3, Gas6 oder Komplementfaktor B bindendes Peptid, umfasst.
